Europäisches Patentamt

**European Patent Office**

**Office européen des brevets**

(19)

(11) Veröffentlichungsnummer: **0 306 541 B1**

(12)

# EUROPÄISCHE PATENTSCHRIFT

(45) Veröffentlichungstag der Patentschrift: **08.01.92**

(51) Int. Cl.⁵: **C07H 15/252**

(21) Anmeldenummer: **87110159.8**

(22) Anmeldetag: **14.07.87**

(54) Verfahren zur Gewinnung von Daunorubicinhydrochlorid aus Fermentbrühe.

(43) Veröffentlichungstag der Anmeldung:
**15.03.89 Patentblatt 89/11**

(45) Bekanntmachung des Hinweises auf die
Patenterteilung:
**08.01.92 Patentblatt 92/02**

(84) Benannte Vertragsstaaten:
**AT CH DE ES FR GB GR LI NL SE**

(56) Entgegenhaltungen:
**EP-A- 0 100 075**
**FR-A- 2 538 394**
**US-A- 4 198 480**

(73) Patentinhaber: **BIOGAL GYOGYSZERGYAR**
**Pallagi ut 13**
**H-4042 Debrecen(HU)**

(72) Erfinder: **Balini, Janos, Dr., Dipl.-Chem.**
**Pechy u. 5**
**H-4032 Debrecen/Ungarn(HU)**
Erfinder: **Emri, Zsuzsanna, Dr., Dipl.-Chem.**
**Kartács u. 36**
**H-4032 Debrecen(HU)**
Erfinder: **Fazekas, Jozsef, Dr., Dipl.-Chem.**
**Homokhát u. 16**
**H-4032 Debrecen-Jozsa(HU)**
Erfinder: **Jakab, Attila, Dipl.-Chem.**
**Sántha K. u. 10**
**H-4032 Debrecen(HU)**
Erfinder: **Toth, György, Dr., Dipl.-Chem.**
**Arany J. u. 3**
**H-4400 Nyiregyháza(HU)**

(74) Vertreter: **Hansen, Bernd, Dr.rer.nat. et al**
**Hoffmann, Eitle & Partner Patentanwälte Arabellastrasse 4 Postfach 81 04 20**
**W-8000 München 81(DE)**

## Beschreibung

Die Erfindung betrifft ein verbessertes Verfahren zur Gewinnung von Daunorubicin-hydrochlorid aus Fermentbrühe durch kombinierte Anwendung von Flüssigkeitsextraktion und Säulenchromatographie.

Daunorubicin ist ein gegen Krebs wirksames Antibiotikum, das erste der Antibiotika vom Anthracyclintyp, das klinisch angewendet wurde. Es wird von Bakterien der Gattung Streptomyces gebildet. Italienische, französische und sowjetische Forscher isolierten das Antibiotikum unabhängig voneinander in der Mitte der Sechziger Jahre (belgische Patentschriften Nr. 639 897 und 632 391; Antibiotiki 11, 763 /1966/).

Die Daunorubicin produzierenden Stämme erzeugen während der Fermentierung zahlreiche Verbindungen, die dem Daunorubicin chemisch nahestehen. Deshalb sind die bis heute zur Isolierung und Reinigung des Daunorubicins bis heute bekanntgewordenen Verfahren außerordentlich kompliziert und teuer. Die reine Substanz wird im allgemeinen durch mehrmalige, mit unterschiedlichen organischen Lösungsmitteln vorgenommene Extraktion oder durch Kombination von Extraktions- und Sorptionsmethoden gewonnen (Biotechnology of Industrial Antibiotics, Ed. van Damme E.J., Marcel Dekker, New York /1984/, pp. 569-594; Advances in Biotechnological Processes, Vol. 3, Ed. A. Mizrahi - Al van Wezel, Alan R. Liss. Inc. New York /1984/, pp. 141-161).

Gemäß der belgischen Patentschrift Nr. 639-897 wird die Fermentbrühe in Gegenwart eines Filtrierhilfsstoffes filtriert. Das rohe Daunorubicin, ein noch stark verunreinigtes Produkt, wird getrennt aus dem mit dem Filtrierhilfsstoff vermischten Mycel und aus der filtrierten Fermentbrühe isoliert, und zwar durch eine mehrstufige Extraktion mit n-Butanol beziehungsweise Chloroform. Zur Reinigung des Rohproduktes wird eine Gegenstromextraktion in n-Butanol und Phosphatpuffer vorgenommen. Die Ausbeute des Verfahrens beträgt, bezogen auf den Wirkstoffgehalt der Fermentbrühe, 21 %.

Gemäß einem weiterentwickelten Extraktionsverfahren (Process Biochem. 14,6-11 /1979/) wird die Fermentbrühe mit n-Butanol verrührt und dann filtriert. Die Phasen des Filtrats werden voneinander getrennt, die butanolische Phase wird eingedampft. Der Rückstand wird mit n-Hexan vermischt und mit angesäuertem Wasser extrahiert. Die das Daunorubicin enthaltende wässrige Phase wird mit einem Gemisch aus Aceton und Toluol gewaschen und dann durch Zusatz von n-Butanol das rohe Daunorubicin-hydrochlorid ausgefällt. Das Rohprodukt wird aus einem Methanol/Äthanol/Chloroform-Gemisch umkristallisiert.

Im Falle von mit Sorptionsverfahren kombinierten Extraktionsmethoden wurden als Adsorbentien das schwach saure Kationenaustauscherharz Amberlite IRC-50 (französische Patentschrift Nr. 1 551 195), Kationenaustauscherharz und Aluminiumoxyd (belgische Patentschrift Nr. 632 391) beziehungsweise Kationenaustauscherharz und Silikagel (Folia Microbiol. 22, 275-285 /1977/) verwendet.

Mit dem in FR-2 538 394 beschriebenen Adsorbtionsverfahren wird Daunorubicin in einer Reinheit von 97 % erhalten, was jedoch für eine Verwendung als Arzneimittel noch nicht ausreicht und daher weitere Reinigungsschritte erforderlich macht.

Die bekannten Verfahren haben die gemeinsamen Nachteile, daß unterschiedliche organische Lösungsmittel in beträchtlichen Mengen verwendet werden müssen und die Ausbeuten recht gering sind. Das Arbeiten mit organischen Lösungsmittel macht die Verfahren nicht nur außerordentlich teuer, sondern erfordert eine Reihe von Maßnahmen auf dem Gebiet des Gesundheitsschutzes, des Umweltschutzes usw., wodurch die Verfahren weiter verteuert werden.

Es ist bekannt, daß Daunorubicin selbst auch kanzerogen und cardiotoxisch ist, d.h. gründliche Arbeitsschutzmaßnahmen erforderlich sind. Diese sind um so schwieriger zu realisieren, je komplizierter, vielstufiger das Verfahren ist.

Ziel der Erfindung war es, ein Verfahren auszuarbeiten, mit dem das Daunorubicin in einfacher und wirtschaftlicher Weise aus der Fermentbrühe gewonnen und und weiter bis zu einer für pharmakologische Zwecke genügenden Reinheit gereinigt werden kann.

Es wurde nun gefunden, daß die Anzahl der Reinigungsschritte bedeutend vermindert werden kann, wenn man in das Isolierverfahren einen säulenchromatographischen Schritt einbaut, bei dem als Adsorbens Silikagel geeigneter Teilchengröße und als Elutionsmittel ein Ameisensäure enthaltendes Chloroform/Methanol-Gemisch verwendet wird.

Die vorstehende Aufgabe wird gelöst durch ein Verfahren zur Gewinnung von Daunorubicin-hydrochlorid aus Fermentbrühe zum Extrahieren mit einem nicht wassermischbaren Lösungsmittel, Eindampfen des Extraktes, Ausfällen des Rohproduktes und Reinigen des Rohproduktes auf chromatographischem Wege, und ist dadurch gekennzeichnet, daß die chromatographische Reinigung an einer als Adsorbens Silikagel enthaltenden Säule mit einem Ameisensäure enthaltenden Gemisch aus Chloroform und Methanol als Elutionsmittel vorgenommen wird, die das Daunorubicin-hydrochlorid enthaltenden Fraktionen eingedampft werden und das Produkt gewünschtenfalls umkristallisiert wird.

Besonders vorteilhaft ist es, wenn das Elutionsmittel 0,2 - 2 Vol.-% Ameisensäure enthält und aus Chloroform und Methanol im Verhältnis 80:20 - 90:10 besteht.

Im folgenden wird das erfindungsgemäße Verfahren ausführlicher erläutert.

Die gegebenenfalls vorher filtrierte Kulturbrühe wird unter schwach alkalischen Bedingungen mit n-Butanol extrahiert. Der Extrakt wird im Vakuum eingedampft und aus dem erhaltenen Rückstand der Wirkstoff in Form seines Hydrochlorids ausgefällt. Das auf diese Weise erhaltene Rohprodukt enthält neben Daunorubicin noch zahlreiche nicht identifizierte Anthracyclinglycoside und Aglycone. Durch einmalige einfache Säulenchromatographie wird aus dem stark verschmutzten Rohprodukt reines Daunorubicin-hydrochlorid gewonnen. Zu diesem Zweck wird das Rohprodukt in der geringstmöglichen Menge Methanol gelöst, und die Lösung wird auf eine Säule aufgebracht, die Silikagel von einer Teilchengröße von vorzugsweise 0,063-0,2 mm. Für die Schärfe der Trennung ist es vorteilhaft, wenn das Silikagel in Form einer mit Chloroform bereiteten Suspension in die Säule eingebracht wird. Das dazu verwendete Chloroform darf weder Methanol noch sonstige Lösungsmittel enthalten. Als Elutionsmittel finden ein Ameisensäure enthaltendes Gemisch aus Chloroform und Methanol Verwendung, zum Beispiel ein Chloroform/Methanol/Ameisensäure-Gemisch im Verhältnis 82:18:1. Das Eluat wird fraktionsweise aufgefangen, und die Fraktionen werden dünnschichtchromatographisch untersucht. Die das Daunorubicin-hydrochlorid enthaltenden Fraktionen sind scharf von den anderen Fraktionen getrennt. Die das Zielprodukt enthaltenden Fraktionen werden eingedampft. Der Eindampfrückstand wird ein einziges Mal umkristallisiert, das dabei anfallende Daunorubicin-hydrochlorid ist genügend rein für pharmakologische Zwecke. Auf den Antibiotikumgehalt der Fermentbrühe bezogen beträgt die Ausbeute 60-62 %. Derartige Ausbeuten werden mit keinem der bekannten Verfahren erzielt.

Die Erfindung wird an Hand der folgenden Beispiele näher erläutert,ist jedoch nicht auf diese Beispiele beschränkt.

Beispiel 1

Zu 500 1 Fermentbrühe (Wirkstoffgehalt: 40 µg/ml (40$\gamma$/ml)) werden bei Raumtemperatur 10 Liter 2 %ige Oxalsäure gegeben. Die Fermentbrühe wird 45 Minuten lang gerührt, dann mit 1 % Filtrierperlit versetzt und weitere 30 Minuten lang gerührt. Dann wird das Mycel abfiltriert. Zur Gewinnung des Antibiotikums aus dem Mycel wird dieses mit 100 1 2 %iger Oxalsäure 30 Minuten lang unter Rühren ausgelaugt und dann filtriert.

Die vereinigten Filtrate (530 l) werden mit 40 %iger Natronlauge auf pH 8,0-8,4 eingestellt und dann mit je 200 Liter n-Butanol zweimal extrahiert. Die n-Butanolphase wird mit 100 1 ionenfreiem Wasser gewaschen, mit n Salzsäure auf pH 5,1-5,5 eingestellt und im Vakuum bei 45 °C eingedampft. Zu dem Eindampfkonzentrat (4,5 Liter) werden 90 ml ionenfreies Wasser gegeben. Dann wird der pH-Wert mit 6 n äthanolischer Salzsäure auf 1,2-1,4 eingestellt. Das Gemisch wird bei 45 °C auf 3/4 seines Volumens eingedampft und dann bei 5 °C 24 Stunden lang kristallisieren gelassen. Das rohe Daunorubicin-hydrochlorid (21 g, Wirkstoff: 60 %) wird abfiltriert, mit n-Butanol gewaschen und im Vakuum bei 25 °C getrocknet. Die Mutterlauge wird erneut auf 3/4 ihres Volumens eingedampft und erneut bei 5 °C zum Kristallisieren aufgestellt. Nach 24 Stunden werden weitere 7 g Produkt (Wirkstoffgehalt: 58 %) abgetrennt und auf die beschriebene Weise gewaschen und getrocknet.

Die vereinigten Rohproduktfraktionen (28 g) werden säulonchromatographisch gereinigt. Dazu werden 5 Liter Silikagel der Teilchengröße 0,063-0,2 mm in Chloroform suspendiert und in eine Säule (Durchmesser:Höhe = 1:20) gefüllt. Das Rohprodukt wird in 124 ml Methanol gelöst und auf die Säule aufgebracht. Dann wird als Elutionsflüssigkeit ein im Verhältnis 82:18:1 bereitetes Gemisch aus Chloroform, Methanol und Ameisensäure auf die Säule geleitet (Geschwindigkeit: 800 ml/h). Es werden Fraktionen zu je 800 ml aufgefangen und dünnschichtchromatographisch untersucht. Die das Daunorubicin enthaltenden, nur einen Fleck gebenden Fraktionen werden vereinigt (12 l) und im Vakuum bei 35 °C auf 1/100 ihres Volumens eingedampft. Das Konzentrat wird bei 5 °C 24 Stunden stehengelassen. Dann werden die Daunorubicin-hydrochlorid-Kristalle abfiltriert, mit Chloroform gewaschen und im Vakuum bei 25 °C getrocknet. Man erhält 12,5 g Produkt, was bezogen auf den Gesamtwirkstoffgehalt der Fermentbrühe einer Ausbeute von 62 %.

| Elementaranalyse für $C_{27}H_{29}NO_{10}HCl$ (M = 563,5) | | | | |
|---|---|---|---|---|
| berechnet, %: | C 57,50 | H 5,32 | N 2,48 | Cl 6,30 |
| gefunden, %: | C 57,10 | H 5,40 | N 2,51 | Cl 6,36 |

3

Schmelzpunkt: 188-189 ° C (Zersetzung)
Dünnschichtchromatographie: auf Merck-Kieselgel 60 , das mit 1 %iger Oxalsäure imprägniert ist, als Laufmittel ein Gemisch aus n-Butanol, Essigsäure und Wasser im Verhältnis 4:1:5 ist $R_f = 0,4$

UV-Spektrum:     $E_{1\ cm}^{1\ \%}$ in Methanol gelöst

| | |
|---|---|
| 233 nm | 620 |
| 250 nm | 440 |
| 286 nm | 138 |
| 476 nm | 201 |
| 494 nm | 199 |
| 532 nm | 115 |

Beispiel 2

500 l Fermentbrühe werden bei pH 8-8,4 (mit 40 %iger Natronlauge eingestellt) mit 400 l n-Butanol extrahiert, ohne daß vorher das Mycel abfiltriert wurde. Die Phasen werden voneinander getrennt, und die organische Phase wird mit dem halben Volumen inonenfreiem Wasser gewaschen. Die gewaschene organische Phase wird auf die im Beispiel 1 angegebene Weise aufgearbeitet. Ausbeute und Parameter des Produktes sind die gleichen wie in Beispiel 1.

**Patentansprüche**

1. Verfahren zur Gewinnung von Daunorubicin-hydrochlorid aus Fermentbrühe durch Extrahieren mit einem nicht wassermischbaren Lösungsmittel, Eindampfen des Extraktes, Ausfällen des Rohproduktes und Reinigen des Rohproduktes auf chromatographischem Wege, dadurch gekennzeichnet, daß die chromatographische Reinigung an einer als Adsorbens Silikagel enthaltenden Säule mit einem Ameisensäure enthaltenden Gemisch aus Chloroform und Methanol als Elutionsmittel vorgenommen wird, die das Daunorubicin-hydrochlorid enthaltenden Fraktionen eingedampft werden und das Produkt gewünschtenfalls umkristallisiert wird.

2. Verfahren nach Anspruch 1, dadurch gekennzeichnet, daß das Elutionsmittel 0,2-2 Vol.-% Ameisensäure enthält und aus Chloroform und Methanol im Verhältnis 80:20 - 90:10 besteht.

**Claims**

1. Process for obtaining daunorubicin hydrochloride from enzyme liquor by extraction with a water-immiscible solvent, concentration of the extract by evaporation, precipitation of the crude product and chromatographic purification of the crude product, characterised in that the chromatographic purification is performed on a column containing silica gel as adsorbent with a formic acid-containing mixture of chloroform and methanol as eluting agent, the fractions containing daunorubicin hydrochloride are concentrated by evaporation, and the product is recrystallised if desired.

2. Process according to Claim 1, characterised in that the eluting agent contains 0.2-2 % by vol. of formic acid and consists of chloroform and methanol in the ratio 80:20 - 90:10.

**Revendications**

1. Procédé d'obtention de chlorhydrate de daunorubicine à partir d'un bouillon de fermentation, par extraction avec un solvant non miscible avec l'eau, concentration par évaporation de l'extrait, précipitation du produit brut et purification du produit brut par chromatographie, caractérisé en ce que la purification chromatographique est réalisée sur une colonne contenant du gel de silice comme adsorbant, avec un mélange contenant de l'acide formique et composé de chloroforme et de méthanol, comme éluant, on concentre par évaporation les fractions contenant le chlorhydrate de daunorubicine et si on le souhaite, on recristallise le produit.

2. Procédé selon la revendication 1, caractérisé en ce que l'éluant contient 0,2 à 2 % en volume d'acide formique et est composé de chloroforme et de méthanol selon un rapport de 80:20 à 90:10.